(19) Europäisches Patentamt
European Patent Office
Office européen des brevets

(11) Veröffentlichungsnummer: **0 011 105**
**B1**

(12) # EUROPÄISCHE PATENTSCHRIFT

(45) Veröffentlichungstag der Patentschrift: **17.02.82**

(21) Anmeldenummer: **79103527.2**

(22) Anmeldetag: **19.09.79**

(51) Int. Cl.³: **C 09 B 48/00,** C 07 D 471/04 // C09B67/00, C08K5/34, (C07D471/00, 221/00, 221/00)

(54) **Bis-N-dialkylcarbamoyl-chinacridone, Verfahren zu ihrer Herstellung und ihre Verwendung.**

(30) Priorität: **29.09.78 DE 2842469**

(43) Veröffentlichungstag der Anmeldung: **28.05.80 Patentblatt 80/11**

(45) Bekanntmachung des Hinweises auf die Patenterteilung: **17.02.82 Patentblatt 82/7**

(84) Benannte Vertragsstaaten: **BE CH DE FR GB**

(56) Entgegenhaltungen:
**DE-A-1 960 896**
**DE-A-1 960 897**
**DE-A-2 148 866**

(73) Patentinhaber: **HOECHST AKTIENGESELLSCHAFT, Zentrale Patentabteilung Postfach 80 03 20, D-6230 Frankfurt/Main 80 (DE)**

(72) Erfinder: **Fuchs, Otto, Dr., Oestricher Weg 11, D-6000 Frankfurt am Main 71 (DE)**
Erfinder: **Kroh, Adolf, Vorderstrasse 27, D-6251 Selters (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 011 105 B1

Bis-N-dialkylcarbamoyl-chinacridone,
Verfahren zu ihrer Herstellung und ihre Verwendung

Gegenstand der Erfindung sind Verbindungen der allgemeinen Formel

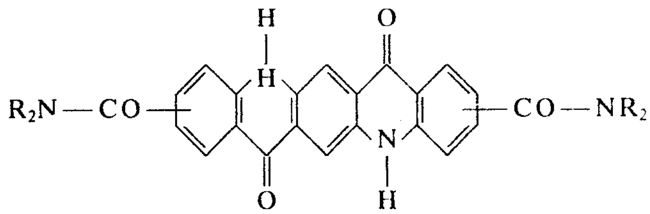

in der R Methyl oder Äthyl ist, sowie Gemische dieser Verbindungen. Bevorzugt steht hierbei die Gruppe $-CO-NR_2$ in 2,9- und 3,10-Stellung.

Aus den DE-AS 1 960 896 und 1 960 897 sind lineare trans-Chinacridone bekannt, die in 2,9-Stellung durch Carbamoyl- oder N-Alkylcarbamoylgruppen mit bis zu 6 Kohlenstoffatomen substituiert sind. Diese Verbindungen stellen trotz der relativ langen Reste Pigmente mit ausgezeichneter Lösemittel-, Ausblut- und Überlackierechtheit dar, was auf die Möglichkeit zurückgeführt wird, daß diese Verbindungen am Carbonamidstickstoff weitere Wasserstoffbrückenbindungen eingehen können. Im Einklang damit zeigen die entsprechenden Dialkylamide, deren Alkylreste 3 und mehr Kohlenstoffatomen haben, eine zunehmende Löslichkeit in organischen Lösemitteln und eine geringere Ausblut- und Überlackierechtheit.

Überraschenderweise nehmen jedoch die ersten beiden Glieder dieser Reihe eine Sonderstellung ein und stellen gelbstichig-rote bis blaustichig-rote Pigmente mit vorzüglichen Echtheiten dar, die denen der Monoalkylamid-Reihe entsprechen.

Gegenüber diesen bekannten Verbindungen zeigen die erfindungsgemäßen Chinacridone jedoch den Vorteil, daß sie im Gemisch mit 50 bis 95 Gew.-% Chinacridon, das durch Methyl oder Chlor substituiert sein kann, bei der Pigmentierung von Lacksystemen außerordentlich transparente Färbungen mit ausgezeichnetem Glanz und einwandfreien rheologischen Eigenschaften ergeben. Solche Gemische, ihre Herstellung und Verwendung als Pigmente sind Gegenstand der europäischen Patentanmeldung Nr. 79 103 528.0

Die erfindungsgemäßen Verbindungen können dadurch hergestellt werden, daß man Verbindungen der allgemeinen Formel

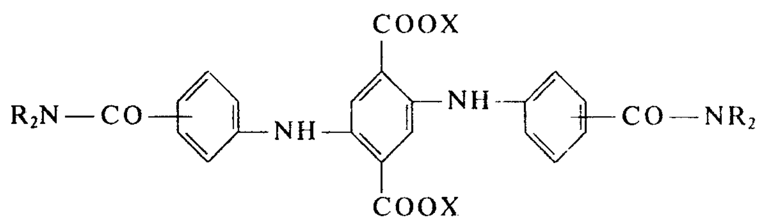

in der R Methyl oder Äthyl ist und X Wasserstoff oder Alkyl mit 1 bis 4 Kohlenstoffatomen bedeutet, in Anwesenheit von sauren Kondensationsmitteln erhitzt.

Die Ausgangsmaterialien können durch Kondensation von Succinylobernsteinsäureestern mit den entsprechenden Aminen, beispielsweise in Alkoholen, anschließende Oxydation, beispielsweise mit Nitrobenzol und gegebenenfalls Verseifung der intermediär gebildeten 2,5-Diarylamino-dihydroterephthalsäureester oder der 2,5-Diarylamino-terephthalsäureester erhalten werden.

Der Chinacridon-Ringschluß erfolgt bevorzugt durch Erhitzen der Diarylaminoterephthalsäuren in sauren Kondensationsmitteln, beispielsweise Polyphosphorsäure, niederen Polyphosphorsäurealkylestern oder Schwefelsäure.

Zur Überführung in eine optimale Pigmentform können die Rohchinacridone — gegebenenfalls nach einem vorherigenAlkaliauszug zur Entfernung von Spuren nicht umgesetzter Diarylaminoterephthalsäuren — bei Temperaturen zwischen 80 und 170° C, gegebenenfalls im geschlossenen Gefäß, einer Behandlung mit Lösemitteln unterworfen werden. Solche Lösemittelbehandlungen sind beispielsweise in der DE-PS 1 261 106 beschrieben. Eine Mahlung der Suspension zur Zerlegung der Agglomerate des Rohchinacridons vor dem Erhitzen kann hierbei vorteilhaft sein.

In den folgenden Beispielen wird die Erfindung näher erläutert. Teile und Prozentangaben beziehen sich hierbei auf das Gewicht. Temperaturen sind in Grad Celsius angegeben.

Beispiel 1

6 Teile 2,5-Bis-(3′-N-dimethylcarbamoyl-phenylamino)-terephthalsäure werden unter Rühren bei 125° in 30 Teile Polyphosphorsäure mit einem Gehalt von ca. 83,5% $P_2O_5$ eingetragen und anschließend 2—3 Stunden bei 125—130° verrührt. Die heiße Schmelze wird dann in Wasser von 50° unter Rühren hydrolysiert. Das isolierte neutrale Rohchinacridon wird in der gleichen Gewichtsmenge 50%igem Äthanol suspendiert und im geschlossenen Gefäß 5 Stunden bei 150° verrührt. Danach wird das Äthanol abdestilliert, das Pigment mit Wasser gewaschen und getrocknet. Das rote Pigment hat gute rheologische Eigenschaften und liefert in Lacken und Kunststoffen Färbungen mit hervorragenden Echtheiten.

Beispiel 2

In 30 Teilen saurem Polyphosphorsäuremethylester ($P_3O_5$-Gehalt ca. 81%) werden unter Rühren bei 100° 5 Teile 2,5-Bis-(4′-N-dimethylcarbamoylphenylamino)-terephthalsäure eingetragen und 2 Stunden bei 125° gerührt. Die Ringschlußschmelze wird in 100 Teilen Wasser von Raumtemperatur hydrolysiert. Das isolierte neutrale, wasserfeuchte Rohchinacridon wird in 30 Teilen Isobutanol (80%) suspendiert und in einem Hochleistungs-Dispergator intensiv gemahlen. Anschließend wird im geschlossenen Gefäß 5 Stunden bei 130° verrührt, danach das Isobutanol mit Wasserdampf abgeblasen, mit Natriumcarbonatlösung auf pH 8,5 gestellt, das Pigment isoliert, neutral gewaschen und getrocknet.

Das rote Pigment ist hervorragend zum Einfärben von Lacken und Kunststoffen geeignet.

Beispiel 3

Verfährt man wie in Beispiel 2 beschrieben, cyclisiert aber 5 Teile 2,5-Bis-(4′-N-diäthylcarbamoyl-phenylamino)-terephthalsäure, so erhält man ein blaustichig-rotes Pigment mit vergleichbaren Eigenschaften.

Im folgenden Beispiel wird der Einsatz eines erfindungsgemäßen Chinacridons im Gemisch mit einem bekannten Chinacridon erläutert, wie es in der europäischen Patentanmeldung Nr. 79 103 528.0 beschrieben ist.

Beispiel 4

10 Teile 2,5-Bis-(4′-methyl-phenylamino)-terephthalsäure und 5 Teile 2,5-Bis-(3′-N-dimethylcarbamoyl-phenylamino)-terephthalsäure werden unter Rühren in 45 Teile Polyphosphorsäure (ca. 83,5% $P_2O_5$) bei 110—120° eingetragen und anschließend 2 Stunden bei 125° verrührt. Die Schmelze wird unter Rühren in 70° heißem Wasser hydrolysiert, das Rohchinacridon abfiltriert und mit Wasser säurefrei gewaschen.

Das Rohchinacridon wird in 75 Teilen Äthanol angerührt, mit Wasser auf insgesamt 150 Teile eingestellt und zweimal über eine Kolloidmühle gemahlen. Anschließend wird 10 Stunden unter Rückfluß gekocht und danach unter gleichzeitigem Zusatz von soviel Wasser, daß das Volumen konstant bleibt, das Äthanol abdestilliert. Bei 60° wird dann die Pigmentsuspension mit Natriumcarbonat schwach alkalisch gestellt, 30 Minuten verrührt, das Pigment abfiltriert, neutral gewaschen und bei 80° getrocknet. Das erhaltene blaustichig-rote Pigmente läßt sich in Lacksystemen sehr gut verarbeiten. Die hochtransparenten Lackierungen zeigen einen hohen Glanz und gute Echtheiten.

Beispiel 5

In 20 Teilen Methylisobutylketon und 10 Teilen Wasser wird das nach Beispiel 2 erhaltene neutrale, wäßrigfeuchte Rohchinacridon suspendiert, die Suspension mit einer Zahnscheibenmühle gemahlen und dann im geschlossenen Gefäß 5 Stunden bei 140° verrührt. Anschließend wird das Methylisobutylketon mit Wasserdampf abdestilliert, die Pigmentsuspension mit Natriumcarbonatlösung auf pH 8,5 gestellt, das Pigment isoliert, neutral gewaschen und getrocknet.

Das Pigment entspricht in seinen coloristischen und Echtheitseigenschaften dem nach Beispiel 2 erhaltenen.

**Patentansprüche**

1. Verbindungen der allgemeinen Formel

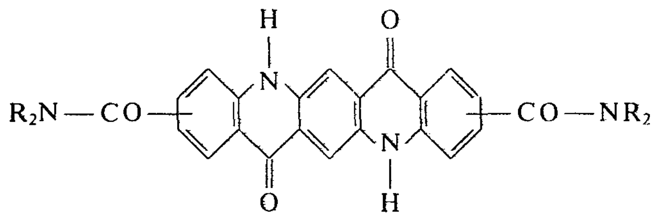

in der R Methyl oder Äthyl ist.

2. Verbindungen nach Anspruch 1, in denen die $-CONR_2$-Gruppen in 2- und 9-Stellung stehen.

3. Verbindungen nach Anspruch 1, in denen die $-CONR_2$-Gruppen in 3- und 10-Stellung stehen.

4. Verfahren zur Herstellung der Verbindungen nach Anspruch 1, dadurch gekennzeichnet, daß man Verbindungen der allgemeinen Formel

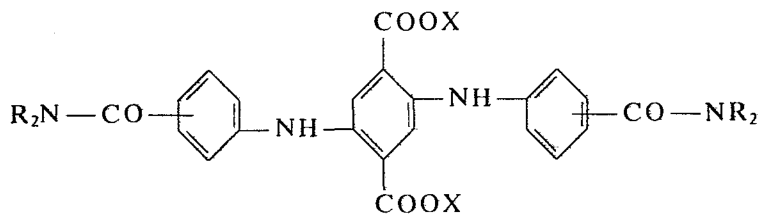

in der R Methyl oder Äthyl ist und X Wasserstoff oder Alkyl mit 1 bis 4 Kohlenstoffatomen bedeutet, in Anwesenheit von sauren Kondensationsmitteln erhitzt.

5. Verfahren nach Anspruch 4, dadurch gekennzeichnet, daß das saure Kondensationsmittel Polyphosphorsäure, ein niederer Polyphosphorsäurealkylester oder Schwefelsäure ist.

6. Verfahren nach Anspruch 4 und 5, dadurch gekennzeichnet, daß das Kondensationsprodukt einer Nachbehandlung mit einem Lösemittel bei Temperaturen von 80 bis 170° C unterworfen wird.

7. Verwendung der Verbindungen nach Anspruch 1 als Pigmente.

8. Verwendung der Verbindungen nach Anspruch 1 zum Pigmentieren von Lacken und Kunststoffen.

**Claims**

1. Compounds of the general formula

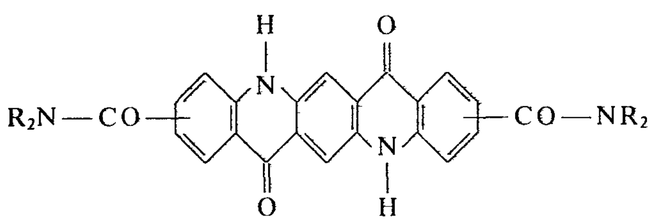

wherein R is methyl or ethyl.

2. Compounds according to claim 1, wherein the groups $-CONR_2$ stand in 2- and 9-position.

3. Compounds according to claim 1, wherein the groups $-CONR_2$ stand in 3- and 10-position.

4. Process for preparing the compounds according to claim 1, characterized by heating compounds of the general formula

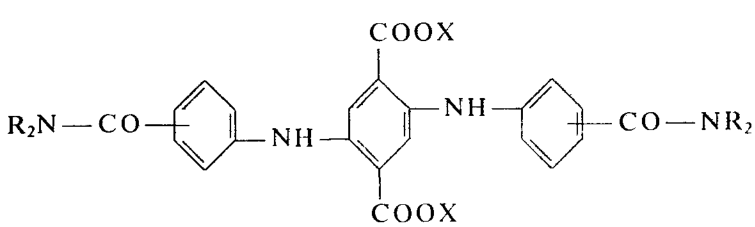

wherein R is methyl or ethyl and X is hydrogen or alkyl with 1 to 4 carbon atoms, in the presence of acidic condensation agents.

5. Process according to claim 4, characterized in that the acidic condensation agent is

polyphosphoric acid, a polyphosphoric acid lower alkyl ester or sulfuric acid.

6. Process according to claims 4 and 5, characterized in that the condensation product is subjected to an aftertreatment with a solvent at temperatures of from 80 to 170° C.

7. Use of the compounds according to claim 1 as pigments.

8. Use of the compounds according to claim 1 for pigmenting paints and plastics.

**Revendications**

1. Les composés de formule générale

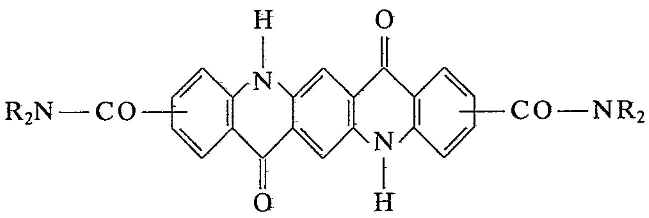

dans laquelle le symbole R représente le groupe méthyle ou éthyle.

2. Composés selon la revendication 1, dans lesquels les groupes $-CONR_2$ occupent les positions 2 et 9.

3. Composés selon la revendication 1, dans lesquels les groupes $-CONR_2$ occupent les positions 3 et 10.

4. Procédé de préparation des composés selon la revendication 1, caractérisé en ce que l'on chauffe avec des agents de condensation acides des composés de formule générale

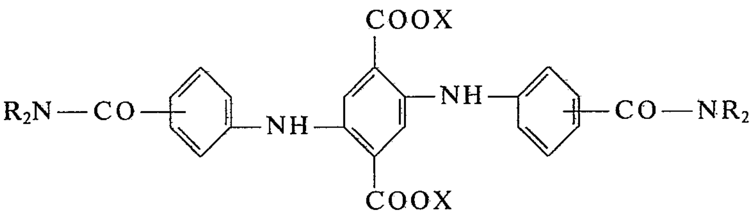

dans laquelle R représente le groupe méthyle ou éthyle et X l'hydrogène ou un alkyle en $C_1$ à $C_4$.

5. Procédé selon la revendication 4, caractérisé en ce que l'agent de condensation acide est de l'acide polyphosphorique, un ester alkylique inférieur de cet acide ou de l'acide sulfurique.

6. Procédé selon la revendication 4 ou 5, caractérisé en ce que le produit de condensation formé est soumis à un traitement ultérieur avec un solvant, à des températures de 80 à 170° C.

7. L'utilisation comme pigments des composés selon la revendication 1.

8. L'utilisation des composés selon la revendication 1, pour pigmenter des vernis, laques et matières plastiques.